# EUROPÄISCHE PATENTSCHRIFT

(11) **EP 1 233 744 B1**
(45) Veröffentlichungstag und Bekanntmachung des Hinweises auf die Patenterteilung: **07.12.2005**
(21) Anmeldenummer: 00993035.5
(22) Anmeldetag: 13.10.2000
(51) Int. Cl.: A61K 7/13

(54) **MITTEL ZUM FÄRBEN VON KERATINHALTIGEN FASERN**
COLOURING AGENT FOR KERATIN CONTAINING FIBRES
AGENT POUR COLORER DES FIBRES A BASE DE KERATINE

(30) Priorität: 22.10.1999 DE 19951010
(43) Veröffentlichungstag der Anmeldung: 28.08.2002
(73) Patentinhaber: Henkel Kommanditgesellschaft auf Aktien, 40589 Düsseldorf (DE)
(72) Erfinder: ROSE, David, 40723 Hilden (DE); HÖFFKES, Horst, 40595 Düsseldorf (DE); MEINIGKE, Bernd, 51381 Leverkusen (DE)
(86) Internationale Anmeldenummer: PCT/EP2000/010074
(87) Internationale Veröffentlichungsnummer: WO 2001/034104

(56) Entgegenhaltungen:
- EP-A- 0 740 931
- DE-A- 4 234 887
- DE-A- 19 707 545

## Beschreibung

Die Erfindung betrifft ein neues Mittel zum Färben von keratinhaltigen Fasern, das eine Kombination aus mindestens einem Diazacycloheptan-Derivat und 4,5-Diamino-1-(β-hydroxyethyl)-pyrazol enthält, sowie ein Verfahren zum Färben von Keratinfasem.

Für das Färben von Keratinfasern, insbesondere menschlichen Haaren, spielen die sogenannten Oxidationsfärbemittel wegen ihrer intensiven Farben und guten Echtheitseigenschaften eine bevorzugte Rolle. Solche Färbemittel enthalten Oxidationsfarbstoffvorprodukte, sogenannte Entwicklerkomponenten und Kupplerkomponenten. Die Entwicklerkomponenten bilden unter dem Einfluß von Oxidationsmitteln oder von Luftsauerstoff untereinander oder unter Kupplung mit einer oder mehreren Kupplerkomponenten die eigentlichen Farbstoffe aus.

Gute Oxidationsfarbstoffvorprodukte müssen in erster Linie folgende Voraussetzungen erfüllen: Sie müssen bei der oxidativen Kupplung die gewünschten Farbnuancen in ausreichender Intensität und Echtheit ausbilden. Sie müssen ferner ein gutes Aufziehvermögen auf die Faser besitzen, wobei insbesondere bei menschlichen Haaren keine merklichen Unterschiede zwischen strapaziertem und frisch nachgewachsenem Haar bestehen dürfen (Egalisiervermögen). Sie sollen beständig sein gegen Licht, Wärme und den Einfluß chemischer Reduktionsmittel, z. B. gegen Dauerwellflüssigkeiten. Schließlich sollen sie - falls als Haarfärbemittel zur Anwendung kommend - die Kopfhaut nicht zu sehr anfärben, und vor allem sollen sie in toxikologischer und dermatologischer Hinsicht unbedenklich sein.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis(2-hydroxyethyl)-p-phenylendiamin, 2-(2',5'-Diaminophenyl)-ethanol, 2-(2,5-Diaminophenoxy)-ethanol, 4-Amino-3-methylphenol, 2-Aminomethyl-4-aminophenol, 2-Hydroxy-4,5,6-triaminopyrimidin, 2,4-Dihydroxy-5,6-diaminopyrimidin, 2,5,6-Triamino-4-hydroxypyrimidin und 1,3-N,N'-Bis(2'-hydroxyethyl)-N,N'-bis(4'-aminophenyl)-diamino-propan-2-ol.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, p-Phenylendiamin, 2,4-Diaminophenoxyethanol, 2-Amino-4-(2'-hydroxyethylamino)-anisol (Lehmanns Blau), 1-Phenyl-3-methyl-pyrazolon-5, 2,4-Dichlor-3-aminophenol, 1,3-Bis-(2',4'-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimiltel, Reformwaren und Körperpflegemittel e.V., Mannheim, Bezug genommen.

In der DE 197 07 545 A1 werden beispielsweise Diazacycloheptan-Derivate beschrieben, die als alleinige Entwicklerkomponenten in Kombination mit bestimmten Kupplern in Haarfärbemitteln eingesetzt werden.

Der Einsatz von 4,5-Diaminopyrazol-Derivaten als Entwicklersubstanzen in Haarfärbemitteln wird beispielsweise in der EP-B-0 740 931 beschrieben. Die Verwendung von weiteren Derivaten ist aus den Druckschriften WO94/08970, WO94/08969 und DE 38 43 892 A1 bekannt.

Allein mit einer Entwicklerkomponente oder einer speziellen Kuppler/Entwickler-Kombination gelingt es in der Regel nicht, eine auf dem Haar natürlich wirkende Farbnuance zu erhalten. In der Praxis werden daher üblicherweise Kombinationen verschiedener Entwicklerkomponenten und Kupplerkomponenten eingesetzt. Es besteht daher ständig Bedarf an neuen, verbesserten Farbstoff-Kombinationen.

Es war daher die Aufgabe der vorliegenden Erfindung, neue Kombinationen von Komponenten zu finden, die geeignet sind, in Färbemitteln eingesetzt zu werden und die insbesondere die an Oxidationsfarbstoffvorprodukte zu stellenden Anforderungen in besonderem Maße erfüllen.

Es wurde nun gefunden, daß Kombinationen aus bestimmten 1,4-Diazacycloheptan-Derivaten und 4,5-Diaminopyrazol-Derivaten sich hervorragend als Färbekomponente in Mitteln zum Färben von keratinhaltigen Fasern eignen und die auch die an Entwicklerkomponenten gestellten Anforderungen in besonders hohem Maße erfüllen. So werden unter Verwendung dieser Entwicklerkomponenten mit den meisten bekannten Kupplerkomponenten brillante Farbnuancen, insbesondere im Braun-, Blau- und Violett-Bereich, erhalten, die außerordentlich licht- und waschecht sind. Weiterhin zeichnen sich die erzielten Färbungen durch außerordentliche Kaltwellechtheit und Wärmestabilität, sowie durch eine hervorragende Egalisierung aus.

Gegenstand der vorliegenden Erfindung sind daher Mittel zum Färben von keratinhaltigen Fasern, die eine Kombination enthalten aus
A) mindestens einem 1,4-Diazacyctoheptan-Derivat der allgemeinen Formel (I) 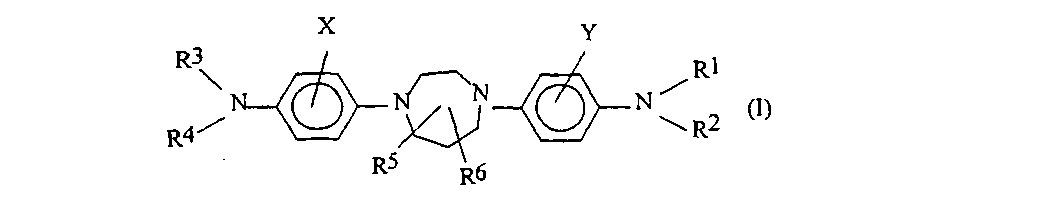 in der
   R¹, R², R³ und R⁴unabhängig voneinander stehen für Wasserstoff, eine C₁₋₄-Alkyl- oder Hydroxyalkylgruppe oder eine C₂₋₄-Dihydroxyalkylgruppe,
   X und Y unabhängig voneinander stehen für Wasserstoff, Chlor, Fluor, eine C₁₋₄-Alkyl-, -Hydroxyalkyl-, -Aminoalkyl- oder -Alkoxygruppe, eine C₂₋₄-Dihydroxyalkylgruppe oder eine Allylgruppe und
   R⁵ und R⁶ unabhängig voneinander stehen für Wasserstoff oder eine C₁₋₄-Alkylgruppe,
   oder einem der physiologisch verträglichen Salze dieser Verbindungen, und
B) 4,5-Diamino-1-(β-hydroxyethyl)-pyrazol und/oder einem der physiologisch verträglichen Salze dieser Verbindung.

Unter keratinhaltigen Fasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten, insbesondere in der Farbphotographie, nichts entgegen.

Die als Komponente A beschriebenen Verbindungen sind beispielsweise aus der DE 197 07 545 A1 bekannt und lassen sich mit bekannten organischen Synthesemethoden herstellen. 4,5-Diaminopyrazole werden beispielsweise in der EP-B-0 740 931 offenbart und lassen sich ebenfalls nach bekannten Synthesemethoden erhalten, sofern sie nicht im Handel erhältlich sind. Die Verbindungen der Komponente A und der Komponente B werden vorzugsweise in einem Molverhältnis von 1 : 20 bis 20 : 1 eingesetzt.

Auch die physiologisch verträglichen Salze lassen sich in an sich bekannter Weise herstellen. Beispiele für solche Salze sind die Hydrochloride, die Hydrobromide, die Sulfate, die Phosphate, die Acetate, die Propionate, die Citrate und die Lactate.

Als Komponente A haben sich die 1,4-Diazacycloheptan-Derivate gemäß Formel (I) erwiesen besonders geeignet erwiesen, bei denen beide Reste R⁵ und R⁶ am 1,4-Diazacycloheptan-Ring Wasserstoff sind.

Ebenfalls erfindungsgemäß bevorzugt sind solche Verbindungen gemäß Formel (I), bei denen mindestens drei, insbesondere alle vier, Gruppen R¹, R², R³ und R⁴ für Wasserstoff stehen.

Schließlich haben sich auch diejenigen 1,4-Diazacycloheptan-Derivate gemäß Formel (I) als erfindungsgemäß besonders geeignet erwiesen, bei denen die beiden Substituenten X und Y an den beiden aromatischen Ringen unabhängig voneinander stehen für Wasserstoff, Fluor, Chlor oder eine C₁₋₄-Alkylgruppe. Wasserstoff sowie Methylgruppen haben sich als ganz besonders vorteilhafte Gruppen X und Y erwiesen.

Besonders hervorragend im Sinne der Erfindung geeignete Substanzen sind N, N'-Bis(4'-aminophenyl)-1,4-diazacycloheptan, N,N'-Bis(4'-amino-2'-methyl-phenyl)-1,4-diazacycloheptan und N,N'-Bis(4'-amino-3'-methyl-phenyl)-1,4-diazacycloheptan.

Unter diesen Verbindungen sind wiederum N,N'-Bis(4'-aminophenyl)-1,4-diazacycloheptan und N,N'-Bis(4'-amino-3'-methyl-phenyl)-1,4-diazacycloheptan bevorzugt.

Die erfindungsgemäßen Mittel eignen sich besonders gut als sogenannte Oxidationsfärbemittel. In Oxidationsfärbemitteln wirkt dieerfindungsgemäße Kombination aus den Verbindungen der Formeln I und II als Entwickler-Komponenten. Es können gewünschtenfalls noch weitere Entwickler-Komponenten sowie Kuppler-Komponenten enthalten sein. Bezüglich der weiteren Entwickler- und Kupplerkomponenten wird auf die zu Beginn der Beschreibung aufgeführten Substanzen verwiesen, die bevorzugte weitere Farbstoffkomponenten darstellen.

Erfindungsgemäß bevorzugte Entwicklerkomponenten sind p-Phenylendiamin, p-Toluylendiamin, p-Aminophenol, o-Aminophenol, 1-(β-Hydroxyethyl)-2,5-diaminobenzol, N,N-Bis-(β-hydroxyethyl)-p-phenylendiamin, 2-(2,5-Diaminophenoxy)-ethanol, 4-Amino-3-methylphenol, 2,4,5,6-Tetraaminopyrimidin, 2-Hydroxy-4,5,6-triaminopyrimidin, 4-Hydroxy-2,5,6-triaminopyrimidin, 2,4-Dihydroxy-5,6-diaminopyrimidin, 2-Dimethylamino-4,5,6-triaminopyrimidin, 2-Hydroxyethylaminomethyl-4-amino-phenol, 4,4'-Diaminodiphenylamin, 4-Amino-3-fluorphenol, 2-Aminomethyl-4-aminophenol, 2-Diethylaminomethyl-4-aminophenol, 2-Hydroxymethyl-4-aminophenol, Bis-(2-hydroxy-5-aminophenyl)-methan, 1,3-Bis-(N-(β-hydroxyethyl)-N-(4'-aminophenyl)amino)-2-propanol sowie 4-Amino-2-(2'-hydroxyethoxy)-phenol.

Ganz besonders bevorzugte weitere Entwicklerkomponenten sind p-Phenylendiamin, p-Toluylendiamin, N,N-Bis-(β-hydroxyethyl)-p-phenylendiamin, 2,4,5,6-Tetraminopyrimidin, 1-(β-Hydroxyethyl)-2,5-diaminobenzol, 3-Methyl-4-aminophenol, o-Aminophenol, 2-Aminomethyl- und 2-Hydroxymethyl-4-aminophenol.

Erfindungsgemäß bevorzugte Kupplerkomponenten sind 1-Naphthol, Pyrogallol, 1,5-, 2,7- und 1,7-Dihydroxynaphthafin, o-Aminophenol, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2',4'-diaminophenoxy)-propan, 4-Chlorresorcin, 2-Chlor-6-methyl-3-aminophenol, 2-Methylresorcin, 5-Methylresorcin, 2,5-Dimethylresorcin, 2,6-Dihydroxypyridin, 2,6-Diaminopyridin, 2-Amino-3-hydroxypyridin, 2,6-Dihydroxy-3,4-diaminopyridin, 3-Amino-2-methylamino-6-methoxypyridin, 4-Amino-2-hydroxytoluol, 2,6-Bis-(2'-hydroxyethylamino)-toluol, 2,4-Diaminophenoxyethanol, 1-Methoxy-2-amino-4-(2'-hydroxyethylamino)-benzol, 2-Methyl-4-chlor-5-amino-phenol, 6-Methyl-1,2,3,4-tetrahydro-chinoxalin, 3,4-Methylendioxyphenol, 3,4-Methylendioxyanilin, 2,6-Dimethyl-3-amino-phenol, 3-Amino-6-methoxy-2-methylaminophenol, 2-Hydroxy-4-aminophenoxyethanol, 2-Methyl-5-(2'-hydroxyethylamino)-phenol und 2,6-Dihydroxy-3,4-dimethylpyridin.

Besonders bevorzugte Kupplerkomponenten sind 1-Naphthol, Resorcin, 1,3-Bis(2',4'-diaminophenoxy)propan, 4-Chlorresorcin, 2,4-Diaminophenoxyethanol, 2-Methylresorcin, 5-Amino-2-methylphenol, 3-Amino-2-chlor-6-methylphenol, 5-Amino-4-chlor-2-methylphenol, 2-Methyl-5-(β-hydroxyethylamino)-phenol, 2-Amino-3-hydroxypyridin, 2-Amino-5-chlor-2-hydroxypyridin, 3-Amino-2-methylamino-6-methoxy-pyridin.

Diese weiteren Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Sulfate, einzusetzen.

Die erfindungsgemäßen Mittel enthalten sowohl die Entwicklerkomponenten der Komponenten A und B als auch die gegebenenfalls vorhandenen Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuß einzelner Oxidationsfarbstoffvorprodukte nicht nachteilig, so daß Entwicklerkomponenten und Kupplerkomponenten in einem Mol-Verhältnis von 1 : 0,5 bis 1 : 3, insbesondere 1:1 bis 1:2, enthalten sein können.

In einer bevorzugten Ausführungsform enthalten die erfindungsgemäßen Mittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, Basic Yellow 57, Disperse Orange 3, HC Red 3, HC Red BN, Basic Red 76, HC Blue 2, HC Blue 12, Disperse Blue 3, Basic Blue 99, HC Violet 1, Disperse Violet 1, Disperse Violet 4, Disperse Black 9, Basic Brown 16 und Basic Brown 17 bekannten Verbindungen sowie 1,4-Bis-(2'-hydroxyethyl)-amino-2-nitrobenzol, 4-Amino-2-nitrodiphenylamin-2'-carbonsäure, 6-Nitro-1,2,3,4-tetrahydrochinoxalin, Hydroxyethyl-2-nitro-toluidin, Pikraminsäure, 2-Amino-6-chloro-4-nitrophenol, 4-Ethylamino-3-nitrobenzoesäure und 2-Chloro-6-ethylamino-1-hydroxy-4-nitrobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Weitere in den erfindungsgemäßen Mitteln enthaltene Farbstoffkomponenten können auch Indole und Indoline, sowie deren physiologisch verträgliche Salze, sein. Bevorzugte Beispiele sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 6-Hydroxyindol, 6-Aminoindol und 4-Aminoindol. Weiterhin bevorzugt sind 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 6-Hydroxyindolin, 6-Aminoindolin und 4-Aminoindolin.

Die erfindungsgemäßen Mittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Mittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Mittel in wasserfreie Träger einzuarbeiten.

Weiterhin können die erfindungsgemäßen Mittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobemsteinsäuremono- und -dialkylester mit 8 bis 18 C-Atomen in der Alkylgruppe und Sulfobemsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Coming 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil®-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methylhydroxyalkyldialkoyloxyalkylammoniummethosulfate.

Ein Beispiel für ein als kationisches Tensid einsetzbares quatemäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wrk-, Hilfs- und Zusatzstoffe sind beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkemmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quatemisierte Proteinhydrolysate,
- Parfümöle, Dimethylisosorbid und Cyclodextrine,
- Lösungsvermittler wie Ethanol, Isopropanol, Ethylenglykol, Propylenglykol. Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Mittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Mittels eingesetzt.

Für das Färbeergebnis kann es vorteilhaft sein, den Mitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Mittels, enthalten.

Zur Herstellung der erfindungsgemäßen Mittel werden die Oxidationsfarbstoffvorprodukte in einen geeigneten wasserhaltigen Träger eingearbeitet. Zum Zwecke der Haarfärbung sind solche Träger z.B. Cremes, Emulsionen, Gele oder auchtensidhaltige schäumende Lösungen, z.B. Shampoos, Schaumaerosole oder andere Zubereitungen, die für die Anwendung auf dem Haar geeignet sind.

Ein weiterer Gegenstand der vorliegenden Erfindung ist ein Verfahren zum Färben von keratinhaltigen Fasern, insbesondere menschlichen Haaren, worin ein Färbemittel, enthaltend eine Kombination aus
A) mindestens einem 1,4-Diazacycloheptan-Derivat der allgemeinen Formel (I) 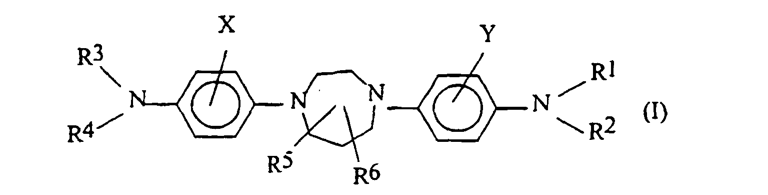 in der
   R¹, R², R³ und R⁴ unabhängig voneinander stehen für Wasserstoff, eine C₁₋₄-Alkyl- oder Hydroxyalkylgruppe oder eine C₂₋₄-Dihydroxyalkylgruppe,
   X und Y unabhängig voneinander stehen für Wasserstoff, Chlor, Fluor, eine C₁₋₄-Alkyl-, -Hydroxyalkyl-, -Aminoalkyl- oder -Alkoxygruppe, eine C₂₋₄-Dihydroxyalkylgruppe oder eine Allylgruppe und
   R⁵ und R⁶ unabhängig voneinander stehen für Wasserstoff oder eine C₁₋₄-Alkylgruppe,
   oder einen der physiologisch verträglichen Salze dieser Verbindungen
B) 4,5-Diamino-1-(β-hydroxyethyl)-pyrazol und/oder einem der physiologisch verträglichen Salze dieser Verbindung.
und/oder Reaktionsprodukten aus diesen Verbindungen sowie üblichen kosmetischen Inhaltsstoffen, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die oxidative Entwicklung der Färbung kann grundsätzlich mit Luftsauerstoff erfolgen. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Weiterhin ist es möglich, die Oxidation mit Hilfe von Enzymen durchzuführen. Dabei können die Enzyme zur Übertragung von Luftsauerstoff auf die Entwicklerkomponente oder zur Verstärkung der Wirkung geringer Mengen vorhandener Oxidationsmittel dienen. Ein Beispiel für ein enzymatisches Verfahren stellt das Vorgehen dar, die Wirkung geringer Mengen (z.B. 1 % und weniger, bezogen auf das gesamte Mittel)Wasserstoffperoxid durch Peroxidasen zu verstärken.

Zweckmäßigerweise wird die Zubereitung des Oxidationsmittels unmittelbar vor dem Haarefärben mit der Zubereitung aus den Oxidationsfarbstoffvorprodukten vermischt. Das dabei entstehende gebrauchsfertige Haarfärbepräparat sollte bevorzugt einen pH-Wert im Bereich von 2 bis 11, insbesondere von 5 bis 10, aufweisen. Besonders bevorzugt ist die Anwendung der HaarMittel in einem schwach alkalischen Milieu. Die Anwendungstemperaturen können in einem Bereich zwischen 15 und 40 °C liegen. Nach einer Einwirkungszeit von ca. 30 Minuten wird das Mittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Die nachfolgenden Beispiele sollen den Erfindungsgegenstand näher erläutern.

### Beispiele

### Ausfärbungen

Es wurde zunächst eine Cremebasis folgender Zusammensetzung hergestellt [alle Angaben sind, soweit nicht anders vermerkt, in g]:

| | |
|---|---|
| Talgfettalkohol | 17,0 |
| Lorol®techn.¹ | 4,0 |
| Texapon®N 28² | 40,0 |
| Dehyton®K³ | 25,0 |
| Eumulgin®B 2⁴ | 1,5 |
| destilliertes Wasser | 12,5 |

| | |
|---|---|
| ¹ C₁₂₋₁₈-Fettalkohol (HENKEL) | |
| ² Natriumlaurylethersulfat (ca. 28 % Aktivsubstanz; CTFA-Bezeichnung: Sodium Laureth Sulfate) (HENKEL) | |
| ³ Fettsäureamid-Derivat mit Betainstruktur der Formel R-CONH(CH₂)₃N⁺(CH₃)₂CH₂COO⁻ (ca. 30 % Aktivsubstanz; CTFA- Bezeichnung Cocoamidopropyl Betaine) (HENKEL) | |
| ⁴ Cetylstearylalkohol mit ca. 20 Mol EO (CTFA-Bezeichnung: Ceteareth-20) (HENKEL) | |

Auf Basis dieser Creme wurde dann folgende Haarfärbecremeemulsion hergestellt:

| | |
|---|---|
| Cremebasis | 50,0 |
| Entwicklerkomponente (A+ B) | 7,5 mmol |
| Kupplerkomponente | 7,5 mmol |
| Na₂SO₃ (Inhibitor) | 1,0 |
| (NH₄)₂SO₄ | 1,0 |
| konz. Ammoniaklösung | ad pH 10 |
| Wasser | ad 100 |

Die Bestandteile wurden der Reihe nach miteinander vermischt. Nach Zugabe der Oxidationsfarbstoffvorprodukte und des Inhibitors wurde zunächst mit konzentrierter Ammoniaklösung der pH-Wert der Emulsion auf 10 eingestellt, dann wurde mit Wasser auf 100 g aufgefüllt.

Die oxidative Entwicklung der Färbung wurde mit Luftsauerstoff, 1 %iger und 9 %iger Wasserstoffperoxidlösung als Oxidationslösung durchgeführt. Hierzu wurde die Emulsion für die Luftoxidation so belassen, für die Oxdiation mit H₂O₂ wurden 100 g der Emulsion mit 50 g Wasserstoffperoxidlösung (1 %ig bzw. 9 %ig) versetzt und vermischt.

Die Färbecreme wurde auf ca. 5 cm lange Strähnen standardisierten, zu 90 % ergrauten, aber nicht besonders vorbehandelten Menschenhaars aufgetragen und dort 30 Minuten bei 32 °C belassen. Nach Beendigung des Färbeprozesses wurde das Haar gespült, mit einem üblichen Haarwaschmittel gewaschen und anschließend getrocknet.
Für die Ausfärbungen wurden folgende Kuppler- und Entwickler-Komponenten verwendet:

### Entwickler-Komponenten

- A: N,N'-Bis(4-aminophenyl)-1,4-diazacycloheptan
- B: 4,5-Diamino-1-hydroxyethylpyrazol

Die Verbindungen A und B wurden in einem Molverhältnis von 1 : 1 eingesetzt.

### Kuppler-Komponenten K

- 1: 4-N,N-Bis-(hydroxyethyl)-amino-2-aminoanisol
- 2: 2-Chlor-6-methyl-3-aminophenol
- 3: 4-Chlor-6-methyl-3-aminophenol
- 4: 2-Amino-3-hydroxypyridin
- 5: 3-(2-Hydroxyethyl)-amino-6-methylphenol
- 6: 3,4-Methylendioxyphenol
- 7: Resorcin
- 8: 2-Methyl-5-aminophenol
- 9: m-Aminophenol
- 10: 2-Methylresorcin
- 11: 2,4-Diaminophenoxyethanol
- 12: 1,3-Bis(2,4-diaminophenoxy)-propan
- 13: 2,6-Dihydroxy-3,4-dimethylpyridin

Es wurden folgende Ausfärbungen gefunden:

| Kuppler | Nuance des gefärbten Haares | | |
|---|---|---|---|
| | Luftoxidation | 1% H₂O₂ | 9 % H₂O₂ |
| K1 | schwarzblau | dunkelviolett | dunkelviolett |
| K2 | graumagenta | dunkelpurpur | dunkelpurpur |
| K3 | braunrot | dunkelrubin | dunkeirubin |
| K4 | purpurgrau | dunkelviolett | dunkelviolett |
| K5 | rotbraun | violettbraun | violettbraun |
| K6 | rehbraun | violettbraun | violettbraun |
| K7 | rotbraun | violettbraun | dunkelrubin |
| K8 | rotbraun | dunkelviolett | dunkelrubin |
| K9 | rotbraun | violettbraun | violettbraun |
| K10 | graurot | violettbraun | violettbraun |
| K11 | dunkelviolett | dunkelviolett | dunkelviolett |
| K12 | schwarzblau | schwarzblau | dunkelviolett |
| K13 | champagner | bronzebraun | bronzebraun |

## Patentansprüche

1. Mittel zum Färben von keratinhaltigen Fasern, die eine Kombination enthalten aus
A) mindestens einem 1,4-Diazacycloheptan-Derivat der allgemeinen Formel (I) 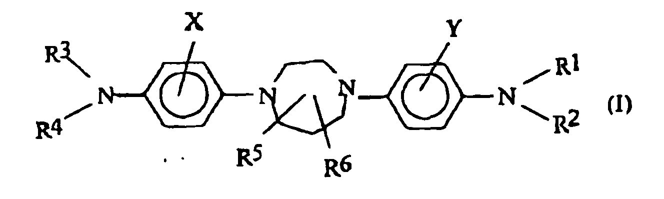 in der
R¹, R², R³ und R⁴ unabhängig voneinander stehen für Wasserstoff, eine C₁₋₄-Alkyl- oder Hydroxyalkylgruppe oder eine C₂₋₄-Dihydroxyalkylgruppe,
X und Y unabhängig voneinander stehen für Wasserstoff, Chlor, Fluor, eine C₁₋₄-Alkyl-, -Hydroxyalkyl-, -Aminoalkyl- oder -Alkoxygruppe, eine C₂₋₄-Dihydroxyalkylgruppe oder eine Allylgruppe und
R⁵ und R⁶ unabhängig voneinander stehen für Wasserstoff oder eine C₁₋₄-Alkylgruppe,
oder einem der physiologisch verträglichen Salze dieser Verbindungen, und
B) 4,5-Diamino-1-(β-hydroxyethyl)-pyrazol und/oder einem der physiologisch verträglichen Salze dieser Verbindung.

2. Mittel nach Anspruch 1, **dadurch gekennzeichnet, dass** in der Verbindung I beide Reste R⁵ und R⁶ für Wasserstoff stehen.

3. Mittel nach Anspruch 1 oder 2, **dadurch gekennzeichnet, dass** in der Verbindung mit der Formel I mindestens drei der Gruppen R¹, R², R³ und R⁴ für Wasserstoff stehen.

4. Mittel nach einem der Ansprüche 1 bis 3, **dadurch gekennzeichnet, dass** in der Verbindung mit der Formel 1 X und Y unabhängig voneinander stehen für Wasserstoff, Fluor, Chlor oder eine C₁₋₄-Alkyl-, insbesondere Methylgruppe.

5. Mittel nach Anspruch 1, **dadurch gekennzeichnet, dass** sie aus N,N'-Bis(4'-aminophenyl)-1,4-diazacycloheptan und N,N'-Bis(4'-amino-3'-methyl-phenyl)-1,4-diazacycloheptan ausgewählt sind.

6. Mittel nach einem der Ansprüche 1 bis 5, **dadurch gekennzeichnet, dass** Verbindungen mit der Formel I und das 4,5-Diamino-1-(β-hydroxyethyl)-pyrazol insgesamt in einer Menge von 0,005 bis 20 Gew.-%, vorzugsweise 0,1 bis 5 Gew.-% enthalten sind.

7. Mittel nach einem der Ansprüche 1 bis 6 **dadurch gekennzeichnet, dass** es ein Oxidationshaarfärbemittel ist.

8. Mittel nach einem der Ansprüche 1 bis 7, **dadurch gekennzeichnet, dass** weitere Entwickler-Komponenten ausgewählt aus 2,4,5,6-Tetraaminopyrimidin, 4-Hydroxy-2,5,6-triaminopyrimidin, 1-(β-Hydroxyethyl)-2,5-diaminobenzol, p-Phenylendiamin, p-Toluylendiamin, p-Aminophenol, 3-Methyl-p-aminophenol, 2-Aminomethyl-p-aminophenol, 2-Diethylaminomethyl-4-aminophenol, N,N-Bis-(β'-hydroxyethyl)-p-phenylendiamin und N,N'-Bis(2'-hydroxyethyl)-N,N'-bis(4'-amino-phenyl)-1,3-diamino-propan-2-ol enthalten sind.

9. Mittel nach einem der Ansprüche 1 bis 8, **dadurch gekennzeichnet, daß** es 1-Naphthol, Resorcin, 1,3-Bis(2',4'-diaminophenoxy)propan, 4-Chlorresorcin, 2,4-Diaminophenoxyethanol, 2-Methyl-resorcin, 5-Amino-2-methylphenol, 3-Amino-2-chlor-6-methylphenol, 5-Amino-4-chlor-2-methylphenol, 2-Methyl-5-(β-hydroxyethylamino)-phenol, 2-Amino-3-hydroxypyridin, 2-Amino-5-chlor-2-hydroxypyridin, 3-Amino-2-methylamino-6-methoxy-pyridin als Kuppler-Komponenten enthält.

10. Mittel nach einem der Ansprüche 8 oder 9, **dadurch gekennzeichnet, dass** die Entwickler- und Kupplerkomponenten jeweils in einer Menge von 0,005 bis 20 Gew.-%, vorzugsweise 0,1 bis 5 Gew.-%, jeweils bezogen auf das gesamte Oxidationsfärbemittel, enthalten sind.

11. Mittel nach einem der Ansprüche 1 bis 10, **dadurch gekennzeichnet, dass** weiterhin mindestens ein direktziehender Farbstoff enthalten ist.

12. Verfahren zum Färben von keratinhaltigen Fasern, insbesondere menschlichen Haaren, worin ein Färbemittel, enthaltend eine Kombination aus
A) mindestens einem 1,4-Diazacycloheptan-Derivat der allgemeinen Formel (I) 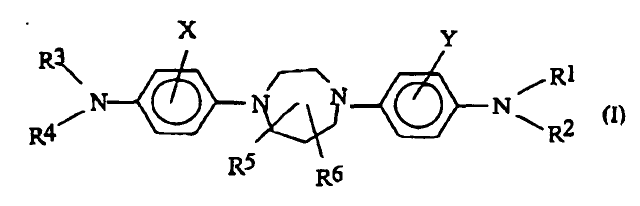 in der
R¹, R², R³ und R⁴ unabhängig voneinander stehen für Wasserstoff, eine C₁₋₄-Alkyl- oder Hydroxyalkylgruppe oder eine C₂₋₄-Dihydroxyalkylgruppe,
X und Y unabhängig voneinander stehen für Wasserstoff, Chlor, Fluor, eine C₁₋₄-Alkyl-, -Hydroxyalkyl-, -Aminoalkyl- oder -Alkoxygruppe, eine C₂₋₄-Dihydroxyalkylgruppe oder eine Allylgruppe und
R⁵ und R⁶ unabhängig voneinander stehen für Wasserstoff oder eine C₁₋₄-Alkylgruppe.
oder einem der physiologisch verträglichen Salze dieser Verbindungen,
B) 4,5-Diamino-1-(β-hydruxyethyl)-pyrazol und/oder einem der physiologisch verträglichen Salze dieser Verbindung
und/oder Reaktionsprodukten aus diesen Verbindungen sowie üblichen Kosmetischen Inhaltsstoffen, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

## Claims

1. Colouring agents for keratin containing fibres which comprise a combination of
A) at least one 1,4-diazacycloheptane derivative of the general formula (I) 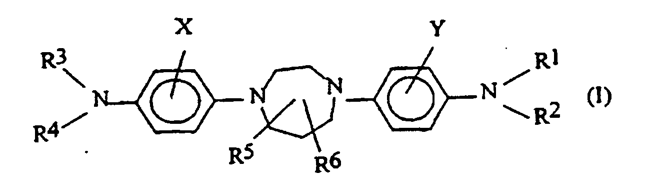 in which
R¹, R², R³ and R⁴, independently of one another, are hydrogen, a C₁₋₄-alkyl or hydroxyalkyl group or a C₂₋₄-dihydroxyalkyl group,
X and Y, independently of one another, are hydrogen, chlorine, fluorine, a C₁₋₄-alkyl, -hydroxyalkyl, -aminoalkyl or -alkoxy group, a C₂₋₄-dihydroxyalkyl group or an allyl group and
R⁵ and R⁶, independently of one another, are hydrogen or a C₁₋₄-alkyl group,
or one of the physiologically compatible salts of these compounds, and
B) 4,5-diamino-1-(β-hydroxyethyl)pyrazole and/or one of the physiologically compatible salts of this compound.

2. Agent according to Claim 1, **characterized in that** in compound I both radicals R⁵ and R⁶ are hydrogen.

3. Agent according to Claim 1 or 2, **characterized in that** in the compound with the formula I at least three of the groups R¹, R², R³ and R⁴ are hydrogen.

4. Agent according to one of Claims 1 to 3, **characterized in that** in the compound with the formula I X and Y, independently of one another, are hydrogen, fluorine, chlorine or a C₁₋₄-alkyl, in particular methyl, group.

5. Agents according to Claim 1, **characterized in that** they are chosen from N,N'-bis(4'-aminophenyl)-1,4-diazacycloheptane and N,N'-bis(4'-amino-3'-methylphenyl)-1,4-diazacycloheptane.

6. Agent according to one of Claims 1 to 5, **characterized in that** compounds with the formula I and 4,5-diamino-1-(β-hydroxyethyl)pyrazole are present in total in an amount of from 0.005 to 20% by weight, preferably 0.1 to 5% by weight.

7. Agent according to one of Claims 1 to 6, **characterized in that** it is an oxidation hair colorant.

8. Agent according to one of Claims 1 to 7, **characterized in that** further developer components chosen from 2,4,5,6-tetraaminopyrimidine, 4-hydroxy-2,5,6-triaminopyrimidine, 1-(β-hydroxyethyl)-2,5-diaminobenzene, p-phenylenediamine, p-tolylenediamine, p-aminophenol, 3-methyl-p-aminophenol, 2-aminomethyl-p-aminophenol, 2-diethylaminomethyl-4-aminophenol, N,N-bis(β'-hydroxyethyl)-p-phenylenediamine and N,N'-bis(2'-hydroxyethyl)-N,N'-bis(4'-aminophenyl)-1,3-diaminopropan-2-ol are present.

9. Agent according to one of Claims 1 to 8, **characterized in that** it comprises 1-naphthol, resorcinol, 1,3-bis(2',4'-diaminophenoxy)propane, 4-chlororesorcinol, 2,4-diaminophenoxyethanol, 2-methylresorcinol, 5-amino-2-methylphenol, 3-amino-2-chloro-6-methylphenol, 5-amino-4-chloro-2-methylphenol, 2-methyl-5-(β-hydroxyethylamino)-phenol, 2-amino-3-hydroxypyridine, 2-amino-5-chloro-2-hydroxypyridine, 3-amino-2-methylamino-6-methoxypyridine as coupler components.

10. Agent according to one of Claims 8 and 9, **characterized in that** the developer and coupler components are present in each case in an amount of from 0.005 to 20% by weight, preferably 0.1 to 5% by weight, in each case based on the total oxidation colorant.

11. Agent according to one of Claims 1 to 10, **characterized in that** at least one direct dye is also present.

12. Method of colouring keratin containing fibres, in particular human hair, in which a colorant comprising a combination of
A) at least one 1,4-diazacycloheptane derivative of the general formula (I) 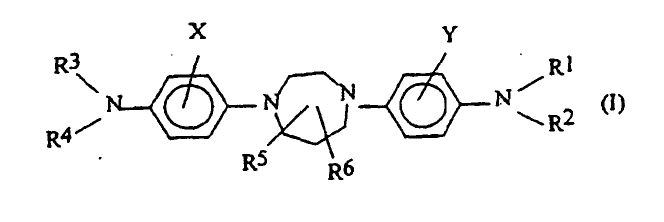 in which
R¹, R², R³ and R⁴, independently of one another, are hydrogen, a C₁₋₄-alkyl or hydroxyalkyl group or a C₂₋₄-dihydroxyalkyl group,
X and Y, independently of one another, are hydrogen, chlorine, fluorine, a C₁₋₄-alkyl, -hydroxyalkyl, -aminoalkyl or -alkoxy group, a C₂₋₄-dihydroxyalkyl group or an allyl group and
R⁵ and R⁶, independently of one another, are hydrogen or a C₁₋₄-alkyl group,
or one of the physiologically compatible salts of these compounds,
B) 4,5-diamino-1-(β-hydroxyethyl)pyrazole and/or one of the physiologically compatible salts of this compound
and/or reaction products from these compounds, and customary cosmetic ingredients is applied to the keratin containing fibres, left on the fibres for a certain time, usually about 30 minutes, and then rinsed out again or washed out with a shampoo.

## Revendications

1. Agents pour colorer des fibres kératiniques, qui contiennent une combinaison de
A) au moins un dérivé de 1,4-diazacycloheptane de formule générale (I) 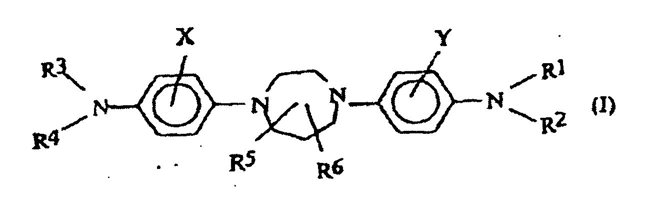 dans laquelle
R¹, R², R³ et R⁴ représentent indépendamment l'un de l'autre un hydrogène, un groupe alkyle ou hydroxyalkyle en C₁ à C₄ ou un groupe dihydroxyalkyle en C₂ à C₄,
X et Y représentent indépendamment l'un de l'autre un hydrogène, un chlore, un fluor, un groupe alkyle, hydroxyalkyle, aminoalkyle ou alcoxy en C₁ à C₄, un groupe dihydroxyalkyle en C₂ à C₄ ou un groupe allyle et
R⁵ et R⁶ représentent indépendamment l'un de l'autre un hydrogène ou un groupe alkyle en C₁ à C₄,
ou l'un des sels physiologiquement acceptables de ces composés, et de
B) 4,5-diamino-1-(β-hydroxyéthyl)-pyrazole et/ou l'un des sels physiologiquement acceptables de ce composé.

2. Agents selon la revendication 1, **caractérisés en ce que** dans le composé I les deux radicaux R⁵ et R⁶ représentent un hydrogène.

3. Agents selon la revendication 1 ou 2, **caractérisés en ce que** dans le composé de formule I au moins trois des groupes R¹, R², R³ et R⁴ représente un hydrogène.

4. Agents selon l'une des revendications 1 à 3, **caractérisés en ce que** dans le composé de formule I X et Y représentent indépendamment l'un de l'autre un hydrogène, un fluor, un chlore ou un groupe alkyle en C₁ à C₄, en particulier un groupe méthyle.

5. Agents selon la revendication 1, **caractérisés en ce qu'**ils sont choisis parmi le N,N'-bis(4'-aminophényl)-1,4-diazacycloheptane et le N,N'-bis(4'-amino-3'-méthylphényl)-1,4-diazacycloheptane.

6. Agents selon l'une des revendications 1 à 5, **caractérisés en ce qu'**ils contiennent des composés de formule I et le 4,5-diamino-1-(β-hydroxyéthyl)-pyrazole au total en une quantité de 0,005 à 20 % en poids, de préférence de 0,1 à 5 % en poids.

7. Agent selon l'une des revendications 1 à 6, **caractérisé en ce qu'**il s'agit d'un agent de coloration des cheveux par oxydation.

8. Agent selon l'une des revendications 1 à 7, **caractérisé en ce qu'**il contient d'autres composants de développement choisis parmi la 2,4,5,6-tétraaminopyrimidine, la 4-hydroxy-2,5,6-triaminopyrimidine, le 1-(β-hydroxyéthyl)-2,5-diaminobenzène, la p-phénylènediamine, la p-toluylènediamine, le p-aminophénol, le 3-méthyl-p-aminophénol, le 2-aminométhyl-p-aminophénol, le 2-diéthylaminoéthyl-4-aminophénol, la N,N-bis-(β'-hydroxyéthyl)-p-phénylènediamine et le N,N'-bis(2'-hydroxyéthyl)-N,N'-bis(4'-amino-phényl)-1,3-diamino-propan-2-ol.

9. Agent selon l'une des revendications 1 à 8, **caractérisé en ce qu'**il contient le 1-naphtol, la résorcine, le 1,3-bis(2',4'-diaminophénoxy)propane, la 4-chlororésorcine, le 2,4-diaminophénoxyéthanol, la 2-méthyl-résorcine, le 5-amino-2-méthylphénol, le 3-amino-2-chloro-6-méthylphénol, le 5-amino-4-chloro-2-méthylphénol, le 2-méthyl-5-(β-hydroxy-éthylamino)-phénol, la 2-amino-3-hydroxypyridine, la 2-amino-5-chloro-2-hydroxypyridine, la 3-amino-2-méthylamino-6-méthoxy-pyridine comme composants de couplage.

10. Agent selon l'une des revendications 8 ou 9, **caractérisé en ce que** les composants de développement et de couplage sont contenus respectivement en une quantité de 0,005 à 20 % en poids, de préférence de 0,1 à 5 % en poids, toujours par rapport à l'ensemble du colorant par oxydation total.

11. Agent selon l'une des revendications 1 à 10, **caractérisé en ce qu'**il contient en outre au moins un colorant montant directement sur la fibre.

12. Procédé de coloration de fibres à base de kératine, en particulier de cheveux humains, dans lequel on dépose sur les fibres kératinqiues un colorant contenant une combinaison de
A) au moins un dérivé de 1,4-diazacycloheptane de formule générale (I) 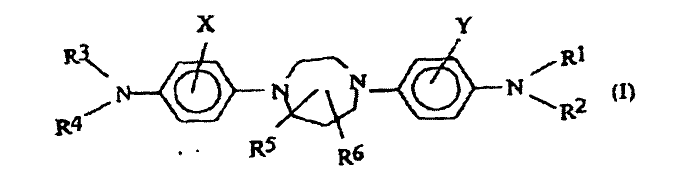 dans laquelle
R¹, R², R³ et R⁴ représentent indépendamment l'un de l'autre un hydrogène, un groupe alkyle ou hydroxyalkyle en C₁ à C₄ ou un groupe dihydroxyalkyle en C₂ à C₄,
X et Y représentent indépendamment l'un de l'autre un hydrogène, un chlore, un fluor, un groupe alkyle, hydroxyalkyle, aminoalkyle ou alcoxy en C₁ à C₄, un groupe dihydroxyalkyle en C₂ à C₄ ou un groupe allyle et
R⁵ et R⁶ représentent indépendamment l'un de l'autre un hydrogène ou un groupe alkyle en C₁ à C₄,
ou l'un des sels physiologiquement acceptables de ces composés,
B) 4,5-diamino-1-(β-hydroxyéthyl)-pyrazole et/ou l'un des sels physiologiquement acceptables de ce composé
et/ou des produits de réaction de ces composés ainsi que des constituants cosmétiques habituels, on le laisse sur les fibres pendant quelque temps, habituellement pendant environ 30 minutes, puis il est de nouveau rincé ou éliminé par lavage avec un shampoing.
